(19) Europäisches Patentamt
European Patent Office
Office européen des brevets

(11) Veröffentlichungsnummer: **0 059 241 B1**

(12) # EUROPÄISCHE PATENTSCHRIFT

(45) Veröffentlichungstag der Patentschrift: **29.01.86**

(51) Int. Cl.[4]: **C 07 C 143/70**

(21) Anmeldenummer: **81108481.3**

(22) Anmeldetag: **19.10.81**

(54) **Verfahren zur Herstellung von aromatischen Sulfohalogeniden.**

(30) Priorität: **28.02.81 DE 3107700**

(43) Veröffentlichungstag der Anmeldung: **08.09.82 Patentblatt 82/36**

(45) Bekanntmachung des Hinweises auf die Patenterteilung: **29.01.86 Patentblatt 86/5**

(84) Benannte Vertragsstaaten: **AT BE CH DE FR GB IT LI NL SE**

(56) Entgegenhaltungen:
**DE - C - 859 461**

**CHEMISCHE BERICHTE, 90. Jahrgang, Nr. 6, 1957, Seiten 841-852, Deutsche Chemische Gesellschaft, H. MEERWEIN et al.: "Verfahren zur Herstellung aromatischer Sulfonsäurechloride, eine neue Modifikation der sandmeyerschen Reaktion"**
**HOUBEN-WEYL: Methoden der organischen Chemie, Band IX: Schwefel-, Tellur- und Selen-Verbindungen. IVe Auflage, Georg Thieme Verlag, Stuttgart, DE. II. Einwirkung von Oxydationsmitteln auf Arylsulfinsäuren. Seiten 332-333**

(73) Patentinhaber: **BASF Aktiengesellschaft, Carl-Bosch-Strasse 38, D-6700 Ludwigshafen (DE)**

(72) Erfinder: **Tonne, Peter, Dr., Triftbrunnenweg 63, D-6730 Neustadt (DE)**
Erfinder: **Jaedicke, Hagen, Dr., Budapester Strasse 49, D-6700 Ludwigshafen (DE)**

**EP 0 059 241 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft ein neues Verfahren zur Herstellung von aromatischen Sulfohalogeniden durch Umsetzung von wässrigen Lösungen aromatischer Diazoniumsalze mit Schwefeldioxid.

Aus der Arbeit von Meerwein et al. (Chem. Ber., Band 90, 1957, S. 841 ff) ist es bekannt, diese Verbindungsklasse durch Diazotierung der entsprechenden aromatischen Amine, Umsetzung der Diazoniumsalzlösungen mit Lösungen von $SO_2$ in Essigsäure und anschliessende oder gleichzeitige Zersetzung der Diazoniumsalze mittels Kupferkatalysatoren wie vor allem Kupferhalogeniden herzustellen, wie am Beispiel der Synthese des Benzolsulfochlorids aus Anilin veranschaulicht sei:

$$C_6H_5NH_2 \xrightarrow[NaNO_2,\ HCl]{Diazotierung} C_6H_5N^{\oplus}{\equiv}N\ Cl^-$$

$$\xrightarrow[Cu_2Cl_2]{SO_2,\ Eisessig} C_6H_5SO_2Cl + N_2$$

Die hierbei erzielbaren Ausbeuten an den Sulfohalogeniden sind jedoch nur in Ausnahmefällen befriedigend, und ausserdem beeinträchtigt der Verbrauch an Essigsäure die Wirtschaftlichkeit des Verfahrens wesentlich. Durch die in der Arbeit von Meerwein et al. empfohlene Mitverwendung eines organischen Lösungsmittels mit niederer Dielektrizitätskonstante, wie Benzol oder Tetrachlorkohlenstoff, lässt sich die Ausbeute im Falle des p-Methoxybenzolsulfochlorids zwar relativ verbessern, lässt jedoch absolut mit nur rd. 40% erheblich zu wünschen übrig.

Der Erfindung lag daher die Aufgabe zugrunde, die aromatischen Sulfohalogenide I in höheren Ausbeuten und auf wirtschaftlichere Weise als bisher herzustellen.

Demgemäss wurde gefunden, dass man aromatische Sulfohalogenide (I) in einer bemerkenswerten Reaktionsfolge durch Umsetzung einer wässrig-halogenwasserstoffsauren Lösung eines aromatischen Diazoniumhalogenides mit Schwefeldioxid erhält, wenn man

a) die wässrige, den Halogenwasserstoff HX enthaltende Lösung eines Diazoniumsalzes, welches sich von dem entsprechenden aromatischen Amin (II) und dem Halogen X des gewünschten Sulfohalogenids ableitet, in Gegenwart eines mit Wasser nicht oder nur begrenzt mischbaren inerten organischen Lösungsmittels mit Schwefeldioxid umsetzt,

b) das Diazoniumsalz gleichzeitig oder anschliessend mittels eines Diazoniumsalz-Zersetzungskatalysators zersetzt und

c) das hierbei erhaltene Reaktionsgemisch oder dessen organische Phase nach Abtrennung der wässrigen Phase mit einem Oxidationsmittel behandelt.

Die Diazoniumsalze können sich prinzipiell von jeglichen diazotierbaren isocyclischen oder heterocyclischen ein- oder mehrkernigen primären aromatischen Aminen II ableiten. Solche Amine, welche beliebige inerte Substituenten tragen können und vorzugsweise der allgemeinen Formel II'

$$Ar(NH_2)_n \qquad II'$$

wobei Ar ein gegebenenfalls substituierter Arylrest und n 1 oder 2 ist, entsprechen, sind beispielsweise die Mono- und Diamine der aromatischen Stammverbindungen Benzol, Naphthalin, Pyridin und Chinolin.

Als inerte Substituenten kommen z.B. in Betracht

- $C_1$–$C_{20}$-Alkylgruppen
- $C_1$–$C_{20}$-Alkoxygruppen
- $C_2$–$C_{20}$-Acylgruppen
- $C_2$–$C_{20}$-Acyloxygruppen
- $C_2$–$C_{20}$-Carbalkoxygruppen
- Carboxylgruppen
- Hydroxylgruppen
- Mono- und Di-$C_1$–$C_4$-alkylaminogruppen
- Halogen wie Fluor, Chlor und Brom
- Halogen- und Hydroxyalkylgruppen
- Nitrogruppen
- Sulfogruppen
- Arylgruppen wie die Phenylgruppe und
- Aralkylgruppen wie die Benzylgruppe

Die Verfahrensprodukte haben demgemäss vorzugsweise die allgemeine Formel I'

$$AR(SO_2X)_n \qquad I'$$

wobei X Halogen, vorzugsweise Chlor, bedeutet.

Die vor dem Reaktionsschritt (a) erforderliche Diazotierung der Amine II ist aus zahllosen Beispielen insbesondere der Farbstoffchemie an sich bekannt und bedarf daher keiner eingehenderen Erläuterung. Im Hinblick auf eine möglichst vollständige Diazotierung und im Hinblick auf das gute Gelingen des erfindungsgemässen Verfahrens hat es sich jedoch als besonders zweckmässig erwiesen, die Diazotierung in halogenwasserstoffsaurem Medium mittels Natriumnitrit bei −10 bis 10°C vorzunehmen, wobei man pro Moläquivalent des Amins 1,5 bis 4 mol HX und 0,2 bis 1 kg Wasser verwendet.

Im Verfahrensschritt (a) lässt man die wässrigsaure, vorzugsweise 10 bis 40 Gew.% des Diazoniumsalzes enthaltende Lösung in Gegenwart eines mit Wasser nicht oder nur begrenzt mischbaren inerten organischen Lösungsmittels mit Schwefeldioxid reagieren.

Zur Erzielung möglichst hoher Ausbeuten an dem Reaktionsprodukt verwendet man zweckmässigerweise mindestens äquimolare Mengen $SO_2$, bezogen auf das Diazoniumsalz, jedoch empfiehlt es sich im allgemeinen, einen molaren $SO_2$-Überschuss bis zu 1 mol, vorzugsweise von etwa 0,1 bis 0,3 mol, einzusetzen. Die Menge des Lösungsmittels beträgt je nach dessen Lösevermögen für das $SO_2$ und das entstehende Sulfochlorid vorzugsweise 0,1 bis 1 l pro Liter der wässrigen Diazoniumsalzlösung.

Die Lösungsmittel können im Prinzip beliebig sein, sofern sie mit Wasser nicht nennenswert mischbar sind, sich gegen wässrige Säuren und $SO_2$ im wesentlichen inert verhalten und ein ausreichendes Lösevermögen für $SO_2$ und das entstehende Sulfochlorid aufweisen.

Die gute Löslichkeit für das Sulfochlorid ist von besonderer Wichtigkeit, so dass sich die Wahl des Lösungsmittels in erster Linie hiernach richtet. Bei dem Erfordernis der geringen Wasserlöslichkeit genügt es dagegen im Prinzip, dass sich eine organische Phase ausbildet. Aus verfahrenstechnischen Gründen ist es jedoch vorteilhaft, ein möglichst wasserunlösliches Lösungsmittel zu verwenden, weil dadurch die im Verfahrenskreislauf erforderliche Trennung des Lösungsmittels von der wässrigen Phase erleichtert wird.

Geeignete Lösungsmittel sind beispielsweise

- aliphatische Ether mit 4 bis 20 C-Atomen wie Diethylether, Di-n-propylether, Diisopropylether, Methylethylether und Methyl-tert.-butylether
- aliphatische Alkohole mit 4 bis 8 C-Atomen wie n-Butanol, n-Pentanol und die Hexanole
- aliphatische Ester mit 2 bis 10 C-Atomen wie Methylacetat, Ethylacetat, n-Propylacetat, Isopropylacetat und die Butylacetate
- aliphatische Ketone mit 4 bis 10 C-Atomen wie Methylethylketon, Diethylketon, Methylisopropylketon und Diisopropylketon
- chlorierte aliphatische Kohlenwasserstoffe mit 1 bis 4 C-Atomen wie Methylenchlorid und die Dichlor- und Trichlorethane und -propane, wobei allgemein solche bevorzugt werden, die bis zu zwei Cl-Atome pro C-Atom enthalten
- aromatische Kohlenwasserstoffe mit 6 bis 10 C-Atomen wie Benzol, Toluol und die Xylole
- chlorierte aromatische Kohlenwasserstoffe wie Chlorbenzol und die Dichlor- und Trichlorbenzole
- Nitrile wie Benzonitril
- Nitroverbindungen wie Nitrobenzol sowie Gemische dieser Lösungsmittel.

Aus wirtschaftlichen und verfahrenstechnischen Gründen werden allgemein solche Lösungsmittel bevorzugt, die bei Normaldruck zwischen 50 und 150°C sieden, da diese bei den im Rahmen des technisch ausgeübten Verfahrens erforderlichen Destillationsschritten am wenigsten Energie verbrauchen. Unter den genannten Gruppen von Lösungsmitteln empfehlen sich besonders die Ketone und die chlorierten aliphatischen Kohlenwasserstoffe, wogegen die aromatischen Lösungsmittel sich als etwas weniger geeignet erwiesen haben.

Man kann die Umsetzung in der Weise vornehmen, dass man eine Lösung aus dem Lösungsmittel und Schwefeldioxid mit der wässrigen Diazoniumsalzlösung oder die drei Komponenten – $SO_2$, Lösungsmittel, wässrige Phase – intensiv miteinander vermischt. In beiden Fällen empfehlen sich Temperaturen von 10–80°C sowie Normaldruck oder ein leicht erhöhter Druck bis zu etwa 6 bar.

Im Verfahrensschritt (b) wird das intermediäre Reaktionsprodukt des Verfahrensschrittes (a) mit Hilfe von Diazoniumsalz-Zersetzungskatalysatoren bei vorzugsweise 20 bis 80°C, besonders 30 bis 60°C, unter Stickstoffentwicklung in dem zweiphasigen Reaktionsgemisch des Verfahrensschrittes (a) zersetzt.

Als Zersetzungskatalysatoren kommen z.B. Kupfer und Wolfram sowie deren Verbindungen in Betracht, die man vorzugsweise in Mengen von 0,005 bis 0,1, besonders 0,01 bis 0,03 mol pro Mol des Amins II in fester Form oder, falls die Katalysatoren wasserlöslich sind, in Form konzentrierter wässriger Lösungen einsetzt. Besonders bevorzugt werden $CuCl_2$ und $Cu_2Cl_2$ als Katalysatoren.

Überraschenderweise eignen sich auch oberflächenaktive quartäre Ammoniumsalze als Zersetzungskatalysatoren in Mengen von etwa 0,01 bis 0,1 mol pro Mol des Amins II. Als besonders geeignet haben sich quartäre Ammoniumsalze des Typs $R\text{-}N^{\oplus}R'_3.X^{\ominus}$ erwiesen, wobei R ein langkettiger Alkylrest, die Reste R′ niedere Alkyl- oder Hydroxyalkylgruppen wie die Methyl- oder Hydroxyethylgruppe und $X^{\ominus}$ ein Anionenäquivalent wie vorzugsweise Chlorid bedeuten. Beispiele für derartige Verbindungen sind Lauryltrimethylammoniumchlorid und Stearyltrimethylammoniumchlorid.

Die Oberflächenaktivität der quartären Ammoniumsalze braucht im übrigen nicht besonders ausgeprägt zu sein. Vielmehr genügt es, wenn diese Salze hinreichend organophil sind wie beispielsweise Tetrabutylammoniumsalze oder Trimethylbenzylammoniumsalze.

Verwendet man Kupfersalze als Zersetzungskatalysatoren, so bewirken oberflächenaktive Verbindungen ganz allgemein eine Beschleunigung der Zersetzung und damit eine Erhöhung der Raum-Zeit-Ausbeute. Die Mengen an diesen Mitteln, die anionisch, nicht-ionisch oder kationisch sein können, betragen vorzugsweise 0,01 bis 0,1 mol pro Mol des eingesetzten Amins II. Auch in diesem Falle empfehlen sich die kationisch oberflächenaktiven quartären Ammoniumsalze, da sie für sich allein bereits als Zersetzungskatalysatoren fungieren, wobei es von besonderem Vorteil ist, dass nur noch bis zu einem Zehntel der Menge an Kupfersalzen erforderlich ist, die man zur Erzielung der gleichen Wirkung mit den Kupfersalzen allein benötigt.

Mit dem freigesetzten Stickstoff entweicht auch ein Teil des Schwefeldioxids, welches in üblicher Weise aus dem Abgasstrom entfernt und wieder in die Verfahrensstufe (b) zurückgeführt werden kann, z.B. durch Wäsche mit dem Lösungsmittel. Der Anteil des zurückgewinnbaren $SO_2$ kann erhöht werden, indem man es nach beendeter Reaktion, also wenn kein Stickstoff mehr entwickelt wird, durch Erwärmen aus dem Reaktionsgemisch austreibt.

In einer alternativen Ausführungsform kann man die Umsetzung mit dem $SO_2$ und dem Katalysator auch gleichzeitig vornehmen. Diese Methode ist in der Regel zu bevorzugen, da sich hierdurch die Raum-Zeit-Ausbeute erhöhen lässt und da wegen kürzerer Gesamtreaktionszeiten weniger Nebenprodukte gebildet werden.

Als Reaktionsprodukt des Verfahrensschrittes (b) erhält man ein wässrig-organisches Gemisch, dessen organische Phase das Sulfochlorid I enthält.

Im Verfahrensschritt (c) lässt man auf dieses Gemisch bei 0 bis 100, vorzugsweise 20 bis 50°C und bei Normaldruck oder leicht erhöhtem Druck - etwa bis zu 6 bar - ein Oxidationsmittel einwirken. Diese Massnahme beruht auf der Beobachtung, dass sich im Reaktionsschritt (b) als Nebenprodukte in aller Regel Sulfinsäuren bilden, und zwar je nach Art des Amins II zwischen etwa 5 und 30 Mol-%, bezogen auf das Amin. Überraschenderweise lassen sich die Sulfinsäuren ohne weiteres zum Sulfochlorid oxidieren, so dass sich die Ausbeute am Sulfochlorid erheblich erhöhen lässt. Im Prinzip eignet sich jedes Oxidationsmittel, jedoch bevorzugt man aus verfahrenstechnischen Gründen Wasserstoffperoxid und freies Halogen, darunter vor allem Chlorgas. Die erforderliche Menge des Oxidationsmittels beträgt etwa 0,05-0,5 mol, in der Regel 0,2-0,3 mol pro Mol eingesetztem Diazoniumsalz. Die Sulfinsäuren können nach der Methode der Hochdruckflüssigkeitschromatographie (HPLC) nachgewiesen werden. In der Regel ist die Oxidation nach etwa 5-30 Minuten beendet.

Anschliessend trennt man die wässrige von der organischen Phase. Die wässrige Phase wird, nachdem man sie in üblicher Weise unter Rückgewinnung des Zersetzungskatalysators aufgearbeitet hat, verworfen.

Man kann die wässrige Phase auch vor der oxidativen Behandlung abtrennen, jedoch empfiehlt es sich in der Regel, diesen Schritt an die Oxidation anzuschliessen, weil die wässrige Phase die wasserlöslichen Nebenprodukte wie u.a. Salze und einen Teil der Sulfinsäure aufnimmt.

Im Anschluss an den Verfahrensschritt arbeitet man das Reaktionsgemisch wie üblich auf die Sulfohalogenide auf. Häufig lässt sich die organische Phase, welche das Sulfohalogenid enthält, auch unmittelbar für weitere Synthesen mit dem Sulfohalogenid verwenden.

Die aromatischen Sulfohalogenide I finden allgemein als Zwischenprodukte für organische Synthesen Verwendung. So werden z.B. Regulatoren für das Pflanzenwachstum und Herbicide des Typs der Arylsulfonylharnstoffe hergestellt, indem man ein aromatisches Sulfohalogenid zunächst in das Sulfamid überführt, dieses mit Phosgen zum Sulfoisocyanat umsetzt, welches seinerseits unter Addition eines Amins das Harnstoffderivat liefert (vgl. z.B. US-PS 4 169 719). Die Wirtschaftlichkeit der Herstellung derartiger Produkte wird durch das erfindungsgemässe Verfahren in aller Regel deutlich erhöht.

Beispiel 1

Herstellung von 2-Chlorbenzolsulfochlorid

100 g 36 gew.%ige Salzsäure (= 1 mol HCl) wurden unter Kühlen zunächst mit 32 g (rd. 0,25 mol) 2-Chloranilin und danach im Laufe von 15 min bei 0-5°C mit 76 g einer 25 gew.%igen Natriumnitritlösung (= 0,28 mol $NaNO_2$) versetzt. Anschliessend wurde die so erhaltene Diazoniumsalzlösung noch 10 min bei 0°C gerührt, wonach die überschüssige salpetrige Säure mit etwas Harnstoff zerstört wurde.

Die Diazoniumsalzlösung wurde sodann bei 20°C unter intensivem Rühren mit einer Lösung aus 100 ml 1,2-Dichlorethan und 21 g (0,33 mol) $SO_2$ in Kontakt gebracht. Danach wurde das Gemisch mit einer konzentrierten wässrigen Lösung von 0,625 g (3,7 mmol) Kupfer-II-chlorid-dihydrat versetzt, unter Rühren auf 50°C erwärmt und solange (80 min) bei dieser Temperatur gehalten, bis kein Stickstoff mehr freigesetzt wurde.

Im Anschluss daran wurden bei 50°C 2,5 g (35 mmol) Chlorgas in das Gemisch eingeleitet. Nach 5 min wurden die Phasen getrennt, und die organische Phase wurde wie üblich auf das Verfahrensprodukt aufgearbeitet. Die Ausbeute an reinem 2-Chlorbenzolsulfochlorid (Sdp. 144-146°C/21 mbar) betrug rd. 93%.

Beispiel 2

Herstellung von 4-Methoxybenzolsulfochlorid

Eine analog Beispiel 1 aus p-Anisidin hergestellte Diazoniumsalzlösung wurde mit einer Lösung aus 100 ml Diethylketon und 21 g (0,33 mol) $SO_2$ in innigen Kontakt gebracht und anschliessend mittels 0,5 g $CuCl_2 \cdot 2H_2O$ und 1 g Tetrabutylammoniumchlorid bei 40°C zersetzt. Die weitere Behandlung mit 4,5 g (63 mol) $Cl_2$ sowie die daran anschliessende übliche Aufarbeitung des Reaktionsgemisches lieferte das 4-Methoxybenzolsulfochlorid in 87%iger Ausbeute; Fp. 43°C.

Beispiel 3

Herstellung von 2-Methoxybenzolsulfochlorid

Eine analog Beispiel 1 aus o-Anisidin hergestellte Diazoniumsalzlösung wurde bei 0°C mit einer Lösung aus 100 ml 1,2-Dichlorethan und 21 g (0,33 mol) $SO_2$ in innigen Kontakt gebracht und anschliessend mittels 0,5 g $CuCl_2$ und 1 g Do-

decyl-dimethyl-benzylammoniumchlorid bei 40°C zersetzt. Danach wurde das Gemisch mit 4,3 g einer 30 gew.%igen wässrigen Wasserstoffperoxidlösung (= 38 mmol $H_2O_2$) versetzt und nach einer Reaktionszeit von 3 min wie üblich aufgearbeitet. Die Ausbeute an 2-Methoxybenzolsulfochlorid betrug 80%; Sdp. 134–138°C/0,7 mbar.

Beispiel 4

Herstellung von 4-Methylbenzolsulfochlorid (Tosylchlorid)

Diese Verbindung wurde analog Beispiel 2 aus p-Toluidin in 74%iger Ausbeute hergestellt; Fp. 67–68°C (Ligroin).

**Patentansprüche**

1. Verfahren zur Herstellung von aromatischen Sulfohalogeniden (I) durch Umsetzung einer wässrig-halogenwasserstoffsauren Lösung eines aromatischen Diazoniumhalogenids mit Schwefeldioxid, dadurch gekennzeichnet, dass man

a) die wässrige, den Halogenwasserstoff HX enthaltende Lösung eines Diazoniumsalzes, welches sich von dem entsprechenden aromatischen Amin (II) und dem Halogen X des gewünschten Sulfohalogenids I ableitet, in Gegenwart eines mit Wasser nicht oder nur begrenzt mischbaren inerten organischen Lösungsmittels mit Schwefeldioxid umsetzt,

b) das Diazoniumsalz gleichzeitig oder anschliessend mittels eines Diazoniumsalz-Zersetzungskatalysators zersetzt und

c) das hierbei erhaltene Reaktionsgemisch oder dessen organische Phase nach Abtrennung der wässrigen Phase mit einem Oxidationsmittel behandelt.

2. Verfahren nach Anspruch 1, dadurch gekennzeichnet, dass man als organisches Lösungsmittel ein aliphatisches Keton mit 4 bis 10 C-Atomen verwendet.

3. Verfahren nach Anspruch 1, dadurch gekennzeichnet, dass man als organisches Lösungsmittel einen chlorierten aliphatischen Kohlenwasserstoff mit 1 bis 4 C-Atomen verwendet, der bis zu zwei Cl-Atome pro C-Atom enthält.

4. Verfahren nach den Ansprüchen 1 bis 3, dadurch gekennzeichnet, dass man als Diazoniumsalz-Zersetzungskatalysator $CuCl_2$ oder $Cu_2Cl_2$ verwendet.

5. Verfahren nach Anspruch 4, dadurch gekennzeichnet, dass man zur Zersetzung des Diazoniumsalzes zusätzlich zu den Kupfersalzen ein oberflächenaktives Mittel mitverwendet.

6. Verfahren nach Anspruch 5, dadurch gekennzeichnet, dass man als oberflächenaktives Mittel ein oberflächenaktives quartäres Ammoniumsalz verwendet.

7. Verfahren nach den Ansprüchen 1 bis 3, dadurch gekennzeichnet, dass man als Diazoniumsalz-Zersetzungskatalysator ein oberflächenaktives quartäres Ammoniumsalz verwendet.

8. Verfahren nach den Ansprüchen 1 bis 7, dadurch gekennzeichnet, dass man als Oxidationsmittel Chlorgas verwendet.

9. Verfahren nach den Ansprüchen 1 bis 7, dadurch gekennzeichnet, dass man als Oxidationsmittel Wasserstoffperoxid verwendet.

**Claims**

1. A process for the preparation of aromatic sulfohalides (I) by reacting an aqueous solution, containing a hydrogen halide, of an aromatic diazonium halide with sulfur dioxide, wherein

a) the aqueous solution, containing the hydrogen halide HX, of a diazonium salt which is derived from the corresponding aromatic amine (II) and the halogen X of the desired sulfohalide (I) is reacted with sulfur dioxide in the presence of an inert organic solvent which is immiscible with water or only miscible with it to a limited extent,

b) the diazonium salt is decomposed, simultaneously or subsequently, by means of a catalyst for diazonium salt decomposition, and

c) the resulting reaction mixture, or the organic phase thereof after removal of the aqueous phase, is treated with an oxidizing agent.

2. A process as claimed in claim 1, wherein the organic solvent used is an aliphatic ketone of 4–10 carbon atoms.

3. A process as claimed in claim 1, wherein the organic solvent used is an aliphatic chlorohydrocarbon of 1–4 carbon atoms, which contains one or two Cl atoms per carbon atom.

4. A process as claimed in claims 1 to 3, wherein $CuCl_2$ or $Cu_2Cl_2$ is used as the catalyst for diazonium salt decomposition.

5. A process as claimed in claim 4, wherein a surfactant is used additionally to the copper salt for decomposing the diazonium salt.

6. A process as claimed in claim 5, wherein a surface-active quaternary ammonium salt is used as the surfactant.

7. A process as claimed in claims 1 to 3, wherein a surface-active quaternary ammonium salt is used as the catalyst for diazonium salt decomposition.

8. A process as claimed in claims 1 to 7, wherein chlorine gas is used as the oxidizing agent.

9. A process as claimed in claims 1 to 7, wherein hydrogen peroxide is used as the oxidizing agent.

**Revendications**

1. Procédé de préparation de sulfo-halogénures (I) aromatiques par réaction d'un halogénure de diazonium aromatique en solution halohydrique aqueuse avec de l'anhydride sulfureux, caractérisé en ce que:

a) on fait réagir une solution aqueuse, contenant de l'hydrogène halogéné HX, d'un sel de diazonium dérivé de l'amine aromatique (II) appropriée et de l'halogène X du sulfo-halogénure I

désiré, en présence d'un solvant organique inerte, non miscible à l'eau ou ne possédant qu'une miscibilité limitée, avec l'anhydride sulfureux;

b) on décompose le sel de diazonium simultanément ou subséquemment à l'aide d'un catalyseur de décomposition des sels de diazonium et

c) on traite le mélange réactionnel obtenu ou la phase organique obtenue après séparation de la phase aqueuse avec un agent oxydant.

2. Procédé suivant la revendication 1, caractérisé en ce que le solvant organique est choisi parmi les cétones aliphatiques en $C_4$ à $C_{10}$.

3. Procédé suivant la revendication 1, caractérisé en ce que le solvant organique est choisi parmi les hydrocarbures aliphatiques chlorés en $C_1$ à $C_4$, comportant jusqu'à deux atomes de chlore par atome de carbone.

4. Procédé suivant les revendications 1 à 3, caractérisé en ce que le catalyseur de décomposition des sels de diazonium est choisi parmi le $CuCl_2$ et le $Cu_2Cl_2$.

5. Procédé suivant la revendication 4, caractérisé en ce que l'on emploie, à côté du sel de cuivre, une substance tensio-active pour la décomposition du sel de diazonium.

6. Procédé suivant la revendication 5, caractérisé en ce que la substance tensio-active est un sel d'ammonium quaternaire tensio-actif.

7. Procédé suivant les revendications 1 à 3, caractérisé en ce que le catalyseur de décomposition des sels de diazonium est un sel d'ammonium quaternaire tensio-actif.

8. Procédé suivant les revendications 1 à 7, caractérisé en ce que l'agent oxydant est du chlore gazeux.

9. Procédé suivant les revendications 1 à 7, caractérisé en ce que l'agent oxydant est du peroxyde d'hydrogène.